Europäisches Patentamt

European Patent Office

Office européen des brevets

(11) Numéro de publication: **0 396 472**
**A1**

## (12) DEMANDE DE BREVET EUROPEEN

(21) Numéro de dépôt: **90401194.7**

(51) Int. Cl.⁵: **A61B 3/02**

(22) Date de dépôt: **03.05.90**

(30) Priorité: **03.05.89 FR 8905884**

(43) Date de publication de la demande:
**07.11.90 Bulletin 90/45**

(84) Etats contractants désignés:
**AT BE CH DE DK ES FR GB GR IT LI LU NL SE**

(71) Demandeur: **OPTIC 2000**
**68, rue de Poitiers, B.P. no 17**
**F-86130 Jaunay-Clan(FR)**

(72) Inventeur: **Mouchet, Bernard**
**La Savine**
**F-39150 St-Laurent du Jura(FR)**

(74) Mandataire: **Kügele, Bernhard**
**NOVAPAT FRANCE 63 bis, boulevard**
**Bessières**
**F-75017 Paris(FR)**

(54) **Dispositif d'évaluation et de contrôle des capacités visuelles.**

(57) La présente invention concerne un dispositif de contrôle et d'évaluation des capacités visuelles mettant en oeuvre une plaque (1) sur laquelle sont imprimés des symboles graphiques et des textes. Le dispositif comporte en outre une lunette de séparation munie d'un filtre vert et d'un filtre rouge.

Application : multi tests de vision de près.

FIG. 1

EP 0 396 472 A1

## DISPOSITIF D'EVALUATION ET DE CONTROLE DES CAPACITES VISUELLES.

La présente invention concerne un dispositif d'évaluation et de contrôle des capacités visuelles mettant en oeuvre au moins deux séries de symboles graphiques ainsi qu'un moyen de séparation tenu devant les yeux du sujet.

On connait dans l'état de la technique des dispositifs de ce type constitués par un boîtier muni d'une source d'éclairage intégrée et d'un écran sur lesquels sont reportées les séries de symboles. Le moyen de séparation est constitué par une paire de lunettes munie de verres polarisés permettant d'affecter à chaque oeil l'une des séries de symboles uniquement.

Les dispositifs connus dans l'état de l'art présentent divers inconvénients. En particulier, le fait d'utiliser une source d'éclairage intégrée rend le dispositif encombrant et couteux. Par ailleurs, la source d'éclairage nécessitant une alimentation électrique, constituée soit par un transformateur pesant et encombrant, soit des piles électriques ou des accumulateurs également lourds et couteux.

L'objet de la présente invention est de remédier à ces inconvénients en proposant un dispositif d'un faible coût de fabrication, d'utilisation simple et ne mettant en oeuvre aucune source d'éclairage intégré. La présente invention concerne plus particulièrement un dispositif d'évaluation et de contrôle des capacités visuelles comportant deux séries de symboles graphiques, les symboles de la première série étant d'une première couleur et les symboles de la deuxième série étant d'une couleur complémentaire, le moyen de séparation comportant un premier filtre présentant un faible facteur de transmission dans la bande de longueur d'onde correspondant à la première couleur et un deuxième filtre présentant un faible facteur de transmission dans la bande de longueur d'onde correspondant à la couleur complémentaire.

Par couleur complémentaire, on entend deux couleurs dont les spectres chromatiques sont suffisamment éloignés pour permettre une séparation facile par deux filtres colorés.

Selon un mode de réalisation préféré, les symboles de la première série sont de couleur verte et les symboles de la deuxième série sont de couleur rouge. Le moyen de séparation est constitué par une paire de lunettes munie d'un filtre rouge et d'un filtre vert.

Selon un premier mode de réalisation, le dispositif de contrôle et d'évaluation des capacités visuelles comporte au moins une paire de symboles identiques, les deux symboles étant légèrement décalés.

Le dispositif selon ce mode de réalisation constitue un test de la stéréoscopie. Ce test, utilisé avec des filtres rouges et verts présente une double disparité pour le couple oculaire, à savoir une disparité de couleur et une disparité de position. Ce test, en particulier lorsqu'il utilise des symboles non usuels et non signifiants, diminue la subjectivité du sujet et permet d'évaluer l'acuité stéréoscopique.

Selon un mode de réalisation préféré, le dispositif selon cette variante comporte plusieurs paire de symboles de formes identiques, de couleurs complémentaires, le décalage entre deux symboles de formes identiques d'une même paire étant différent pour chacune des paires. Le dispositif selon ce mode de réalisation permet de déterminer l'acuité stéréoscopique maximale par la détermination du seuil relatif au-delà duquel le sujet cesse de percevoir l'impression . Cette évaluation apportera à l'opticien ou à l'ophtamologiste des renseignements sur l'équilibre amétropique et sur la qualité de la convergence fusionnelle.

Il est bien entendu que les symboles mis en oeuvre peuvent présenter une forme quelconque, en particulier des cercles, carrés ou toutes autres formes géométriques abstraites ou encore tous objets visuels. Le décalage de deux symboles d'une même paire est calculé selon des règles géométriques bien connue de l'homme du métier.

Selon un deuxième mode de réalisation, le dispositif de contrôle et d'évaluation comporte une première série de symboles constituée par un index et une deuxième série de symboles de couleur complémentaire réalisant une graduation. Par index, on entend tout symbole permettant un repérage face à une série de graduation, en particulier une flèche, un trait ou un triangle. La graduation est avantageusement constituée par une pluralité de repères verticaux centrés sur l'index vertical. Le dispositif selon ce mode de réalisation permet de contrôler l'hétérophorie dans le plan horizontal. Tout défaut visuel du sujet se traduira par une impression de décalage de l'index par rapport aux graduations.

Selon une variante, la graduation est constituée d'une pluralité de repères horizontaux centrés sur un index horizontal.

Le dispositif selon cette variante permet de mesurer l'hétérophorie dans le plan vertical.

Le dispositif selon la présente invention est particulièrement avantageux pour un opticien car il permet d'évaluer immédiatement et de façon extrêmement simple l'efficacité des verres correcteurs proposés au sujet.

Selon un mode de réalisation préféré, le dispositif selon la présente invention comporte en outre au moins deux textes constitués de caractères de taille standardisée. Le dispositif permet ainsi de combiner plusieurs tests d'évaluation de contrôle et en particulier de vérifier l'acuité visuelle au moyen de textes de taille décroissante correspondant aux indices Parinaud.

Selon un mode de réalisation préféré, le dispositif comporte en outre une paire de plages, le fond de l'une des plages étant de la première couleur et le fond de l'autre plage étant de la couleur complémentaire, les plages comportant des caractères identiques de taille identique. Le dispositif ainsi réalisé constitue une batterie de tests complette mettant en outre en évidence les capacités accommodatives d'un sujet et de contrôler, dans le cas d'un presbyte, la valeur de l'addition proposée.

Selon un mode de réalisation préféré, le dispositif de contrôle et d'évaluation comporte en outre un symbole constitué par un carré présentant deux angles opposés de couleur complémentaire, formé de deux segments de droites isochromes et perpendiculaires et deux angles opposés formés de deux segments de droites de couleur complémentaire perpendiculaires, chaque côté étant formé par deux segments de droites de couleur complémentaire, le symbole comportant en outre un point blanc disposé au centre du carré.

Ce dispositif ainsi réalisé est un test d'aniséïconie permettant de vérifier dans quelle mesure le sujet perçoit les variations dimensionnelles créées par les grossissements différents entre l'oeil droit et l'oeil gauche. Le point blanc constitue un élément de fusion évitant les décalages horizontaux et verticaux liés à une éventuelles hétérophorie. Ce test permet d'évaluer les gains éventuels d'un système de lentilles par rapport à un système de lunettes et de savoir si le sujet présente une anisométropie axile ou une anisométropie de puissance.

Selon un mode de réalisation préféré, le dispositif de contrôle et d'évaluation comporte en outre un texte présentant des caractères d'une première couleur, des caractères de la couleur complémentaire, des caractères blancs et des paires de symboles légèrement décalés de couleurs complémentaires.

Les parties de texte en caractères blancs constituent des éléments de fusion évitant le décalage provoqué par les phories horizontales en vision de près. Les paires de symboles décalés doivent être vues en stéréoscopie par le sujet.

Selon un mode de réalisation préféré de l'invention, le dispositif d'évaluation et de contrôle est constitué par une plaque de fond sombre sur lequel sont imprimés les différents symboles, la plaque comportant un logement de moyen de séparation.

La présente invention sera mieux comprise à la lecture de la description qui va suivre, s'appuyant sur les dessins où :

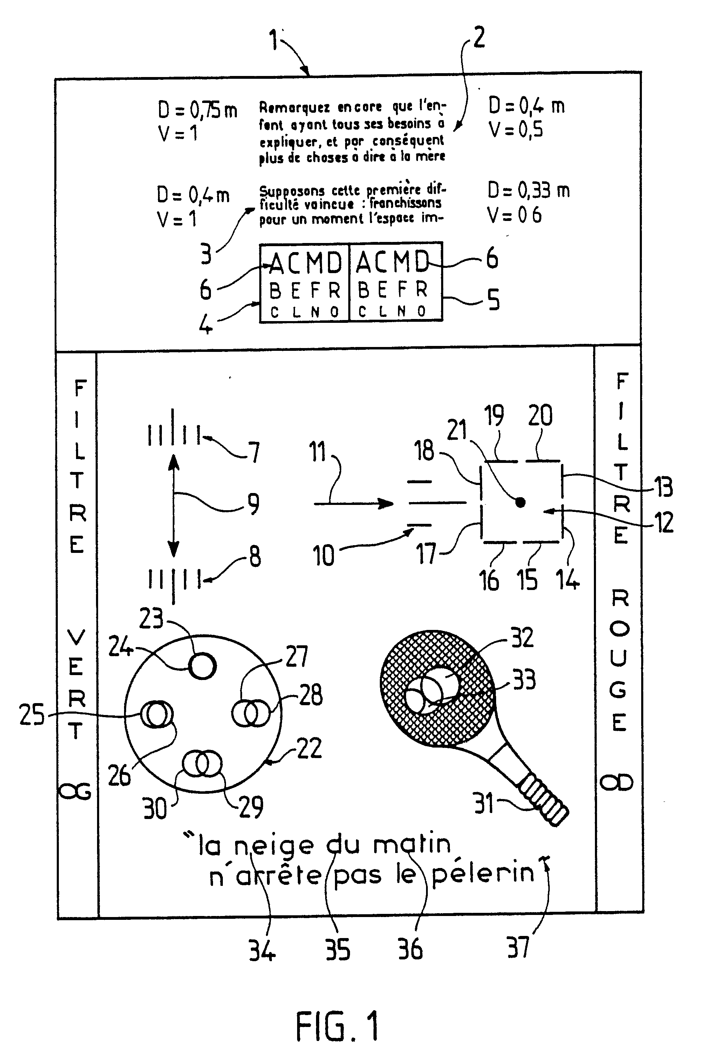

- la figure 1 représente une vue de face d'un dispositif selon la présente invention.

La figure 1 représente une vue d'un dispositif de contrôle et d'évaluation des capacités visuelles en vision de près au niveau :
- de l'accommodation,
- des dimensions d'images,
- des hétérophories,
- des 3 degrés de la vision binoculaire corticale.

Ce dispositif est constitué par une plaque (1) en matière plastique ou en carton présentant un fond noir sur lequel sont imprimés les différents symboles.

Il comprend une première série de symboles constitués par un texte (2) imprimé en lettres blanches sur fond noir et dont la taille des caractères correspond au Parinaud (3).

Un deuxième texte (3) comporte des lettres blanches sur fond noir correspondant au Parinaud (2).

La seconde série de tests est constituée par une première plage colorée en vert (4) et une deuxième plage (5) colorée en rouge. Chacune des plages (4, 5) comporte des symboles (6) permettant de mettre en évidence les capacités accommodatives du sujet à contrôler. Trois types de réponse sont possibles :
- si le sujet considère que les deux plages présentent la même lisibilité, l'équilibre accommodatif est bon pour la distance proposée, ainsi que l'addition dans le cas du sujet presbyte,
- si le sujet à l'impression que la plage rouge domine, il se trouve en position myopique pour la distance proposée, et l'ophtamologue ou l'opticien en déduit que soit l'addition positive est trop forte, soit qu'il existe un problème d'hétérophorie avec une tendance à la sur convergence qui entraîne une sur accommodation.
- si le sujet à l'impression que le vert domine, il se trouve en position hyperopique pour la distance proposée. Il convient dans ce cas de proposer des additions positives et de contrôler la correction hyperopique en vision de loin.

Le dispositif comporte également des symboles constituant des graduations (7, 8) formées de repères verticaux imprimés en rouge associés à une flèche (9) imprimée en vert. Ce test permet d'évaluer l'hétérophorie dans le plan horizontal. A l'aide des filtres verts et rouges disposés devant les yeux du sujet, on supprime la tendance à fusionner. On peut ainsi mettre en évidence l'hétérophorie en vision de près. Les défauts se traduisent par une impression de décalage de la flèche (9) par rapport à la position centrale des graduations (7, 8). Le dispositif comporte un test similaire constitué par une graduation (10) formée par des repères horizontaux associés à une flèche verte horizontale (11). Ce test permet d'évaluer l'hétérophorie dans le plan vertical, et éventuellement de constater un mauvais centrage vertical des verres correcteurs, ou un mauvais positionnement de la lunette.

Le dispositif comporte en outre un test d'aniséïconie constitué par un carré (12) présentant, en partant du coin supérieur droit et en tournant dans le sens trigonométrique, un segment (13) vert, un segment (14) rouge, un segment (15) rouge, un segment (16) vert, un segment (17) rouge, un segment (18) vert, un segment (19) vert, un segment (20) rouge. Un point blanc (21) est disposé au centre du carré ainsi formé. Ce test permet de vérifier dans quelle mesure le sujet perçoit les variations dimensionnelles créées par des grossissements différents entre l'oeil droit et l'oeil gauche. Tout défaut se traduira par une impression de décalage de certains des segments par rapport à d'autres.

Le dispositif comporte également deux tests de stéréoscopie constitué pour le premier par un cercle blanc (22) dans lequel sont inscrits quatre paires (23-24, 25-26, 27-28, 29-30), de couleur complémentaire. Le décalage des cercles de couleur complémentaire est différent pour chacune des paires. Lorsque la vision stéréoscopique est normale, le sujet à l'impression de voir quatre cercles disposés dans des plans en avant de la plaque support (1). Un deuxième test est constitué par un objet signifiant, dans le cas présent une raquette de tennis (31) imprimée en blanc et comportant deux symboles représentant une balle de tennis verte (32) et une balle de tennis rouge (33)

Un test de lecture en vision simultanée est constitué par un texte comportant des mots (34) imprimés en caractères verts, des mots (35) imprimés en caractères blancs et des mots (36) imprimés en caractères rouges. Les guillemets (37) sont constitués de deux symboles de couleur différente et doivent apparaître pour un sujet normal dans un plan en avant de la plaque support (1).

La plaque support (1) présente dans son épaisseur un logement dans lequel peut être glissé une lunette constituée par une monture plane sur laquelle sont collés un filtre vert et un filtre rouge.

Il est bien entendu que la présente invention ne se limite pas au mode de réalisation décrit à titre d'exemple, mais s'étend au contraire à toutes les variantes, en particulier à des dispositifs comportant des symboles de formes, de tailles ou de couleurs différentes.

## Revendications

1 - Dispositif d'évaluation et de contrôle des capacités visuelles, du type mettant en oeuvre deux séries de symboles graphiques ainsi qu'un filtre de séparation tenu devant les yeux du sujet, caractérisé en ce que les symboles de la première série sont d'une première couleur et les symboles de la deuxième série sont d'une deuxième couleur complémentaire, lesdites series de symboles étant inscrites sur un fond noir, le moyen de séparation comportant un premier filtre présentant un faible facteur de transmission dans la bande de longueur d'onde correspondant à la première couleur et un deuxième filtre présentant un faible facteur de transmission dans la bande de longueur d'onde correspondant à la deuxième couleur.

2 - Dispositif d'évaluation et de contrôle des capacités visuelles selon la revendication 1, caractérisé en ce que les symboles de la première série sont de couleur verte et les symboles de la deuxième série sont de couleur rouge, le moyen de séparation étant constitué par une lunette munie d'un filtre coloré rouge et d'un filtre coloré vert.

3 - Dispositif d'évaluation et de contrôle des capacités visuelles selon l'une quelconque des revendications 1 à 2, caractérisé en ce qu'il comporte au moins une paire de symboles identiques, de couleur complémentaire, les deux symboles de la paire étant légèrement décalés.

4 - Dispositif d'évaluation et de contrôle des capacités visuelles selon la revendication 3, caractérisé en ce qu'il comporte plusieurs paires de symboles (23, 24) de forme identique et de couleur complémentaire, le décalage entre deux symboles (23, 24) de forme identique d'une même paire étant différent pour chacune des paires.

5 - Dispositif d'évaluation et de contrôle des capacités visuelles selon l'une quelconque des revendications 1 à 2, caractérisé en ce que la première série de symboles est constituée par un index (9) d'une première couleur et la deuxième série de symboles est constituées par au moins une graduation (7) de couleurs complémentaires.

6 - Dispositif d'évaluation et de contrôle des capacités visuelles selon la revendication 5, caractérisé en ce que la graduation (7) est constituée d'une pluralité de repères verticaux centrés sur un index (9) vertical.

7 - Dispositif d'évaluation et de contrôle des capacités visuelles selon la revendication 5, caractérisé en ce que la graduation (10) est constituée d'une pluralité de repères horizontaux, centrés sur un index horizontal (11).

8 - Dispositif d'évaluation et de contrôle des capacités visuelles selon l'une quelconque des revendications 1 à 7, caractérisé en ce qu'il comporte en outre au moins deux textes (2, 3) constitués de caractères de taille standardisée.

9 - Dispositif d'évaluation et de contrôle des capacités visuelles selon l'une quelconque des revendications 1 à 8, caractérisé en ce qu'il comporte en outre une paire de plages colorées, les fonds des deux plages étant de couleurs complémentaires, les plages comportant des caractères (6) identiques de taille identique.

10 - Dispositif d'évaluation et de contrôle des capacités visuelles selon l'une quelconque des revendications 1 à 9, caractérisé en ce qu'il comporte en outre un symbole constitué par un carré formé par une succession de segments de droites, le premier en commençant par l'un des coins étant d'une première couleur, les deux suivants d'une couleur complémentaire, le suivant de la première couleur, le suivant de la couleur complémentaire, les deux suivants de la première couleur et le dernier de la couleur complémentaire, un point (21) blanc étant disposé au centre dudit carré

11 - Dispositif d'évaluation et de contrôle des capacités visuelles selon la revendication 10, caractérisé en ce qu'il comporte en outre un texte comportant des caractères (34) de la première couleur, des caractères (35) de la deuxième couleur, des caractères blancs (31) et au moins une paire de symboles (37) de couleur complémentaire.

12 - Dispositif d'évaluation et de contrôle des capacités visuelles selon l'une quelconque des revendications 1 à 11, caractérisé en ce qu'il est constitué par une plaque (1) de fond sombre sur laquelle sont imprimés lesdits symboles, ladite plaque comportant un logement du moyen de séparation.

D = 0,75 m
V = 1

Remarquez encore que l'enfant ayant tous ses besoins à
expliquer, et par conséquent
plus de choses à dire à la mère

D = 0,4 m
V = 0,5

D = 0,4 m
V = 1

Supposons cette première difficulté vaincue : franchissons
pour un moment l'espace im-

D = 0,33 m
V = 0 6

```
ACMD   ACMD
B E F R  B E F R
C L N O  C L N O
```

FILTRE VERT ⊗G

FILTRE ROUGE ⊗

"la neige du matin
n'arrête pas le pélerin"

FIG. 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | GB-A-2 180 365  (CAMPBELL MCKAY TAYLOR) * page 1, ligne 86 - page 2, ligne 69; figures 2,3 * | 1,2,9 | A 61 B    3/02 |
| A | --- | 5 | |
| A | SOVIET INVENTIONS ILLUSTRATED semaine 8911, abrégé 89 - 083 267/11 P31 P32, 26 avril 1989, Derwent Publications Ltd., London, GB; & SU-A-1421366 (E.E. SOMOV) 07.09.1988 * résumé * ----- | 1,2,9 | |

DOMAINES TECHNIQUES RECHERCHES (Int. Cl.5)

A 61 B    3/00

Le présent rapport a été établi pour toutes les revendications

| Lieu de la recherche | Date d'achèvement de la recherche | Examinateur |
|---|---|---|
| BERLIN | 26-06-1990 | WEIHS J.A. |